# EUROPEAN PATENT APPLICATION

(11) **EP 0 756 847 A1**
(43) Date of publication of application: **05.02.1997**
(21) Application number: 96305567.8
(22) Date of filing: 30.07.1996
(51) Int. Cl.: A61B 5/00, A61B 5/024

(54) **Securement system**

(30) Priority: 31.07.1995 GB 9515660
(71) Applicant: JOHNSON & JOHNSON MEDICAL, INC., Arlington, Texas 76004-3130 (US)
(72) Inventor: West, Ian, St. Fagans, Cardiff, CF5 4UF (Wales) (GB); Evans, Peter Dilwyn, Penylan, Cardiff, CF3 7ET (Wales) (GB); Eagles, Obreon, Morriston, Swansea, SA6 6DD (Wales) (GB)
(74) Representative: Mercer, Christopher Paul

(57) **Abstract**

A securement system (1), for use with an instrument (16) to be placed adjacent a patient's skin. The securement system (1) has a first member (2) with adhesion means (4) for adhesion to a patient's skin, and a second member (14) which is repeatably attachable to the first member (2). An instrument (16), when placed on said first member (2) with said second member (14) attached to the first member (2), has a fixed position in relation to the first member (2). Apertures (18, 20, 21, 22) are provided in the first member (2) through which the instrument (16) can operate.

## Description

This invention relates to a securement system, for use in securing an instrument to a patient's skin such as a medical sensor, particularly for use with non-invasive electromagnetic radiation systems in which light is directed through a patient's skin to measure characteristics of tissue by the spectral absorbency of constituent substances within the tissue. Although the securement system is particulary for use with a medical sensor, other sensors for industrial or scientific applications may also use such a securement system, as may other, non-sensor, instruments.

Non-invasive clinical investigations are carried out by placing a sensor against a patient's skin and passing electromagnetic radiation from an emitter of the sensor into the patient's tissue. In known sensors, the radiation is scattered and most is absorbed by the tissues, but a small proportion is detected by one or more photodetectors which are placed against the patient's skin a distance away from the emitter. Such sensors may give readings which are affected by light tracking along the surface of the skin, rather than through the tissue, and by light being scattered back out of the skin and then reflected towards the detectors by objects in close proximity to the sensor. These alternative light paths have the disadvantage of adding a large and unpredictable offset to genuine signals from the tissues.

Some sensors can be adversely affected by high levels of ambient light and therefore require fixation to a patient which improves the immunity of the sensor to ambient light.

It is known to place sensors on a patient's skin by use of an adhesive between the sensor and the skin or by use of adhesive pads which are placed between the sensor and the patient's skin. It is also known to incorporate a high light absorbency layer around the sensor's outlets and inlets between the sensor and the skin surface to prevent light tracking between the surfaces or light being scattered out of the skin and reflected back to the detectors.

Such known methods of location of a sensor against a patient's skin have a disadvantage that the sensor can not be removed and replaced mid-operation. If the sensor is removed during its operation, or if the monitoring session is interrupted, for example to perform a medical intervention, then precise relocation of the sensor can not be guaranteed. Consequently, data collected before and after the interruption cannot be directly compared. In addition, the adhesive surface of the sensor quickly becomes worn and loses it's adhesive properties if the sensor is removed and replaced.

In instances where the sensor or other instrument is high cost, the sensor or instrument is required to be reusable.

According to the first aspect of the present invention there is provided a securement system, for use with an instrument to be placed adjacent a patient's skin, comprising a first member with adhesion means for adhesion to a patient's skin, and a second member which is repeatably attachable to said first member and wherein, when an instrument is placed on said first member and said second member is attached to said first member, the position of the instrument is fixed in relation to the first member.

Preferably, the first and second members are repeatably attachable to each other by use of repeatable attachment means.

Preferably, the attachment means between the first member and the second member acts as positioning means for the instrument in relation to the first member. There may be provided an additional layer between one or both of the first and second members and the attachment means.

Preferably, the first member has stable dimensions whilst being adapted to be formed against a surface.

The attachment means between the first member and the second member may substantially surround an area of the first member in which an instrument is placeable.

The first member may be formed of a high absorbency material for absorbing electromagnetic radiation to which the instrument is sensitive, the high absorbency material having apertures for the outlets and inlets of the instrument.

The second member may optimally exert a force on the instrument to hold it in uniform contact with the first member. The second member may be elastic and stretch over the instrument.

Preferably, the attachment means is a Velcro (Registered Trade Mark) material with opposing surfaces disposed on the first and second members.

The first and second members when attached together may be formable to the shape of the surface to which the first member is attached.

The first member may be formed of polyurethane foam with visible light and infrared absorbing pigments. The adhesive means is optimally a thin layer of adhesive which is non-sensitising to the skin.

Straps may be provided for securement of the system around a part of a patient's body.

According to a second aspect to the present invention there is provided in combination an instrument and a securement system comprising a first member with adhesion means for adhesion to a patient's skin, a second member which is repeatably attachable to the first member and an instrument disposed between the first and second members, wherein the instrument is held in fixed position in relation to the first member.

The securement system may comprise any of the preferred features of the first aspect of the invention.

An embodiment of the present invention will now be described, by way of example only, with reference to the accompanying drawings in which:

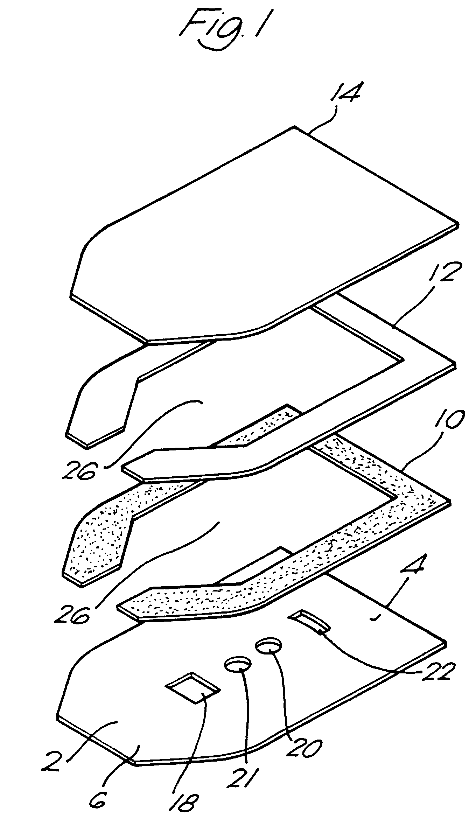

Figure 1 is an exploded perspective view of a securement system in accordance with the present invention;

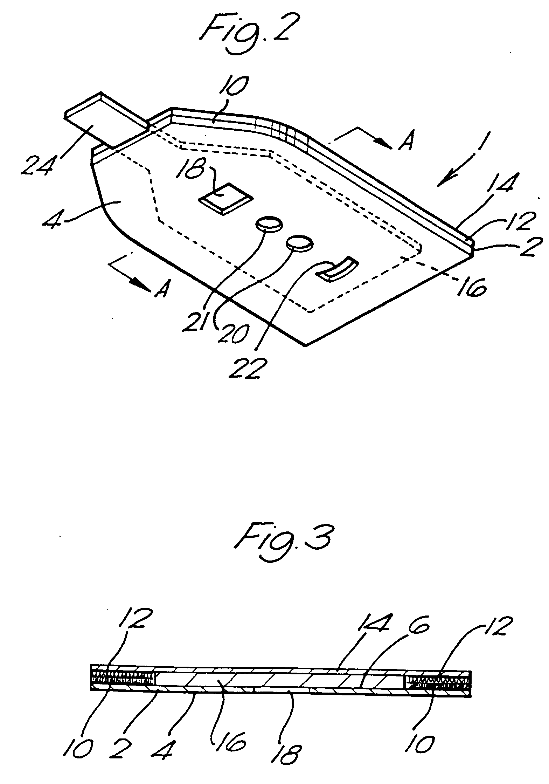

Figure 2 is a perspective view of a securement system in accordance with the present invention;

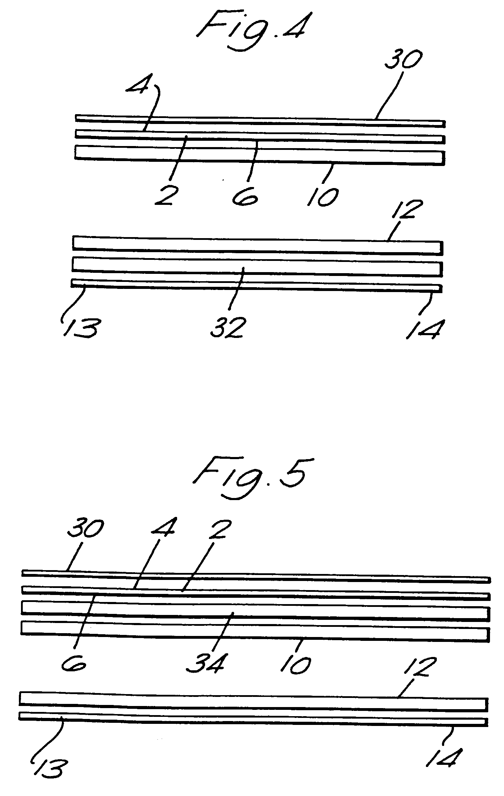

Figure 3 is a cross-section through the line A-A of Figure 2;
Figure 4 is an exploded cross-section of the layers of a securement system in accordance with the present invention with an additional layer in the lower part of the system;

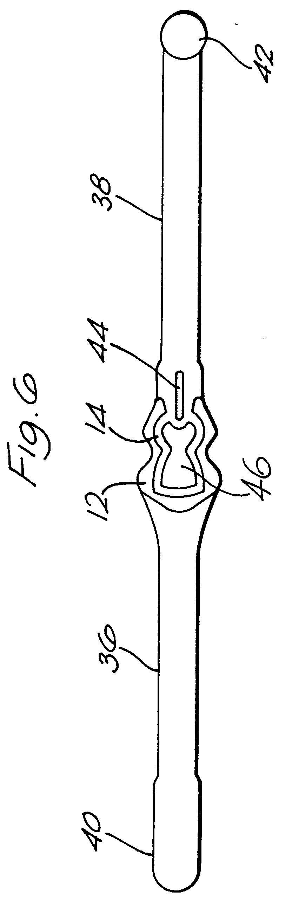

Figure 5 is an exploded cross-section of the layers of a securement system in accordance with the present invention with an additional layer in the upper part of the system; and
Figure 6 is a plan view of a second embodiment of a securement system in accordance with the present invention.

Referring to the drawings, a securement system 1 is provided with a first member 2 with a lower adhesive surface 4 and an upper, non-adhesive, surface 6. An instrument 16 is placeable against the upper surface 6 within the periphery of the first member 2. The first member 2 has a number of apertures 18, 20, 21, 22 through which the instrument 16 can operate. A first portion of an attachment means in the form of a hooked Velcro surface 10 is attached to the upper surface 6 of the first member 2 surrounding the area against which the instrument 16 is placeable. The Velcro surface 10 has an inner profile which corresponds to the profile of the instrument 16 such that the instrument 16 fits snugly within the Velcro surface 10. The hooked Velcro surface 10 has greater dimensional stability and provides a better inner profile than a looped Velcro surface.

A second member 14 is formed of a flexible elastic material and has a profile corresponding to that of the first member 2. The second member 14 is attached to a second portion of the attachment means in the form of a looped Velcro surface 12 which mirrors and is attachable to the hooked Velcro surface 10. The second member 14 and the looped Velcro surface 12 can be slightly smaller than the first member 2 and the hooked Velcro surface 10 to encourage stretching of the second member 14.

The first and second members 2, 14 are formed of 0.4mm thick polyurethane foam. The foam is an open cell structure which is breathable to the skin. An extra layer 32, 34 of 2mm thick polyurethane foam can be placed between either or both the first and second members 2, 14 and the Velcro surfaces 10, 12 to build up the profile of the Velcro surfaces 10, 12. The extra layer 32 between the second member 14 and the Velcro surface 12 is useful when the depth of the instrument 16 is too great for the second member 14 to easily stretch over the instrument 16. The extra layers 32, 34 are also useful if the instrument 16 requires a greater degree of location in the securement system 1.

The shapes of the first and second members 2, 14 and the size of the Velcro surfaces 10, 12 are maximised at strategic points (not shown) around the instrument 16 to ensure that the fixation is secure whilst keeping the overall size of the securement system 1 to a minimum. The size of the Velcro surfaces 10.12 are minimised in other areas.

The first and second members 2, 14, the Velcro surfaces 10, 12 and the extra layers 32, 34 are all adhered together using a thin layer of adhesive. The adhesive layer must be thin to avoid causing light tracking within the securement system 1. The adhesive can be pigmented with carbon black or similar pigment to be absorbing to infrared and visible light.

A protective paper 30 is used to stick to and cover the lower adhesive surface 4 of the first member 2 before this is attached to the patient.

When the two members 2, 14 are attached together by the attachment of the two Velcro surfaces 10, 12, the instrument 16 is held captive between the two members 2, 14. A cable 24 of the instrument 16 may extend from the cavity 26 formed by the first member 2 and the second member 14 through a break in the combined Velcro circumference of the two Velcro surfaces 10, 12. The cavity 26 is deeper if one or both of the extra layers 32, 34 are used.

The apertures 18, 20, 21, 22 in the first member 2 through which the instrument 16 operates can be elongated to ensure that the instrument outlets and inlets line up with the apertures 18, 20,21, 22 with different degrees of curvature of the instrument 16.

The instrument 16, for this example a tissue spectroscopy sensor, has one or more electromagnetic radiation point light sources which direct radiation through the surface of a patient's skin. The light from the one or more sources is detected by photodetectors provided on the instrument 16 a fixed distance from the sources and also adjacent the skin surface. The securement system 1 forms a cavity 26 in which the instrument 16 is enclosed with apertures 18, 20, 21, 22 through which the light sources and detectors operate. The adhesive surface 4 of the first member 2 is adhered to a patient's skin such that the instrument's outlets and inlets, in the form of the sources and detectors, are positioned adjacent the patient's skin. The instrument's outlets and inlets are surrounded by the adhesive surface 4 which is formed of a high electromagnetic radiation absorbency material such as polyurethane foam with absorbent pigments. In this example, the instrument 16 is sensitive to near infrared light and therefore the pigment is an infrared and visible light absorber such as carbon black.

A second embodiment of the securement system 1 is described with reference to Figure 6. This embodiment has been modified for long term monitoring by the addition of straps 36, 38 to the second, upper member 14. The straps 36, 38 provide further securement for long term monitoring where the adhesive properties of the adhesive surface 4 of the first member 2 may gradually degrade due to moisture from the patient's skin.

The straps 36, 38 extend from the second member 14 in opposite directions. The free end 40 of one of the straps 36 has an area of Velcro hooks on its underside. The free end 42 of the other one of the straps 38 has an area of Velcro loops on its upper side. When the straps 36, 38 are passed around a part of a patient such as the patient's head, the Velcro loops and hooks form an attachment. Velcro attachment means has the advantage of being attachable in varying positions enabling a secure fixation of the straps 36, 38 about varying sizes of body portion.

The securement system 1 of the second embodiment has an elongate slot 44 through which the instrument 16 can be passed allowing a cable attached to the instrument 16 to run on the opposite side of the straps 36, 38 from the instrument 16.

The straps 36, 38 help to ensure stable, long term positioning of the instrument 16 but cannot be used in all instances due to space restrictions from other equipment attached to the patient.

An additional localised downforce can be provided over a strategic area of the instrument 16 by an additional spacer member 46 which is disposed between the second member 14 and the instrument 16 within the Velcro surface 12 (see Figure 6). The spacer member 46 can be formed from an area of Velcro loops which would otherwise be waste. It might alternatively be any soft/flexible material such as foam or rubber.

In use, the first member 2 is adhered to the patient's skin and the instrument 16 is placed on the first member 2 within the profile defined by the first Velcro surface 10. The Velcro surface 10 provides dimensional stability for the first member 2. The second member 14 is placed over the instrument 16 and the second Velcro surface 12 is attached to the first Velcro surface 10. The second member 14 is formed of a layer of a flexible elastic material such that the second member 14 is required to be stretched over the instrument 16, and therefore exerts pressure on the instrument 16 holding it in contact on its lower surface with the upper surface 6 of the first member 2.

The use of Velcro as the attachment means has the advantage that when the two surfaces of Velcro 10, 12 are attached together they can take the form (for example, the curvature) of the surface to which the first member 2 is attached. The first member 2 is sufficiently flexible to take the form of the surface to which it is attached. The second member 14 when attached to the first member 2 then takes the same form as the first member 2. Alternative forms of repeatable attachment means can be used with the same results.

The securement system 1 can be disposable for use with one patient only. The first member 2 is adhered to a patient's skin and the instrument 16 is placed in the cavity 26. The second member 14 is placed over the instrument 16 and the operation of the instrument can commence. If the operation is interrupted, the second member 14 can be removed from the first member 2 and the instrument 16 removed from the patient. If the first member 2 remains adhered to the patient's skin, the instrument 16 can be replaced in the cavity 26 of the first member 2 in the same position within the first Velcro surface 10, the second member 14 can be replaced and the operation continued as the instrument 16 will be in exactly the same position against the patient's skin.

Modifications and improvements can be made to the above without departing from the scope of the present invention.

## Claims

1. A securement system (1), for use with an instrument (16) to be placed adjacent a patient's skin, comprising a first member (2) with adhesion means (4) for adhesion to a patient's skin, and a second member (14) which is repeatably attachable to said first member (2) and wherein, when an instrument (16) is placed on said first member (2) and said second member (14) is attached to said first member (2), the position of the instrument (16) is fixed in relation to the first member (2).

2. A securement system (1) as claimed in claim 1, wherein the first and second members (2, 14) are repeatably attachable to each other by use of repeatable attachment means (10, 12).

3. A securement system (1) as claimed in claim 1 or claim 2, wherein the attachment means (10, 12) between the first member (2) and the second member (14) acts as positioning means for the instrument in relation to the first member (2).

4. A securement system (1) as claimed in claim 1 or claim 2, wherein an additional layer (32, 34) is provided between one or both the first and second members (2, 14) and the attachment means (10, 12).

5. A securement system (1) as claimed in any of the preceding claims, wherein the first member (2) has stable dimensions whilst being adapted to be formed against a surface.

6. A securement system (1) as claimed in any of the preceding claims, wherein the attachment means (10, 12) between the first member (2) and the second member (14) substantially surrounds an area of the first member (2) in which an instrument (16) is placeable.

7. A securement system (1) as claimed in any of the preceding claims, wherein the first member (2) is formed of a high absorbency material for absorbing electromagnetic radiation to which the instrument is sensitive, the high absorbency material having apertures (18, 20, 21, 22) for the outlets and inlets of the instrument (16).

8. A securement system (1) as claimed in any of the preceding claims, wherein the second member (14) exerts a force on the instrument (16) to hold it in uniform contact with the first member (12), the second member (14) being elastic and stretching over the instrument (16).

9. A securement system (1) as claimed in any of claims 2 to 8, wherein the attachment means (10, 12) is a Velcro (Registered Trade Mark) material with opposing surfaces disposed on the first (2) and second members (14).

10. A securement system (1) as claimed in any of the preceding claims, wherein the first and second members (2, 14) when attached together are formable to the shape of the surface to which the first member (2) is attached.

11. A securement system (1) as claimed in any of the preceding claims, wherein the first member (2) is formed of polyurethane foam with infrared and visible light absorbing pigments.

12. A securement system (1) as claimed in any of the preceding claims, wherein the adhesive means is a thin layer of adhesive which is non-sensitising to the skin.

13. A securement system (1) as claimed in any of the preceding claims, wherein straps (36, 38) are provided for securement of the system (1) around a part of a patient's body.

14. An instrument (16) and a securement system (1) in combination comprising a first member (2) with adhesion means (4) for adhesion to a patient's skin, a second member (14) which is repeatably attachable to the first member (2) and an instrument (16) disposed between the first and second members (2, 14), wherein the instrument (16) is held in fixed position in relation to the first member (2).

15. An instrument (1) and a securement system (1) in combination as claimed in claim 14, wherein the securement system (1) comprises any of the features of claims 1 to 13.
